# EUROPEAN PATENT APPLICATION

(11) **EP 1 295 938 A1**
(43) Date of publication of application: **26.03.2003**
(21) Application number: 01402435.0
(22) Date of filing: 21.09.2001
(51) Int. Cl.: C12N 15/00, C12N 15/85, C07K 14/61, C12Q 1/68, A61K 38/27, A61K 39/395, A61K 48/00, G01N 33/50, C07K 16/26, C12N 5/00

(54) **Corrected sequence of the hGH-V gene and an allelic variant**

(71) Applicant: GenOdyssee, 91974 Courtaboeuf (FR)
(72) Inventor: Escary, Jean-Louis, 78150 Le Chesnay (FR)
(74) Representative: Rinuy, Santarelli

(57) **Abstract**

The present invention relates to new polynucleotides comprising a SNP in the nucleotide sequence of the hGH-V gene, new polypeptides comprising a mutation caused by this SNP as well as their therapeutic uses.

LISTE DE SEQUENCES

<110> GenOdyssee

<120> New polynucleotides and polypeptides of the hGH-V gene

<130> placental growth hormone

<140>
<141>

<160> 9

<170> PatentIn Ver. 2.1

<210> 1
<211> 5002
<212> ADN
<213> Homo sapiens

<400> 1
tattgaaata gccccaaccc tgggcaccag gagacaggag gctcagtcct ggccttctct 60
gccttcacct tgtcctgctg tgaggtttcc acaaggcacc ccatgccact gggccattct 120
cacctgagaa ttgagaaaga acaatctgcc ccttatggaa agacaaaatg aggcaaaggg 180
ccccttgtgt tgggggcagg atccctgcac tcagagggat atagaagctg aaataactgg 240
gttgggagca gtggctcacg caagtaatct cagtactttg ggaggccagg gagggtggat 300
cacctaaagt caagagttcg agaccagcct ggccaacata gtgaaacccc cgtctctact 360
aaaaaagtgc aaatcttagc ccggcatagt ggtggacgcc tgtaatccca gctacttggg 420
aggctgaggc gagagaatca ccggaaccca aaggtggagg ttgctgtgaa gcaacatcat 480
gccactgaac tccagcctgg gtgacagaat gagactccat ctcaaaaaaa aaaaaaaaag 540
gaaatgaagc taaaataact aaataactga gtagcaccac actacgtgct ctggagaaag 600
gatttttttt tttttttttt ttgagatgta gactcgctct gtcatccagg ctggagtgca 660
gtggcgcaat cttggctcac agcaacctct gcctcctggg ttcaagcgat tctcctgcct 720
cagcctccca agtagctggg attacgggct cgtgccacca tgaccagcta atttttgtat 780
ttttaggaga gatggagttt tgccatgtgg gttagcctgg tcttgcactc ctgaccttaa 840
gtgatccacc cacctcagcc tcccaaagtg ctgggattat aggcatgagc caccgtgccc 900
ggcctggaga aaggacttta aatgacgcaa tgtgggaaga gcaaggttgt gaagatctgc 960
tgccctggct gaggtagctc atgtaatcag tctctctgag cctcagtctc ctgatctgtg 1020
aaatgggatg atagtcatag ctcctacaca agacaagtgg caggtcagac gtgagaatgc 1080
acaggcaggc ccttggcaac tggataagct ctacacaggt ctggaaagga ggagacaaaa 1140
gaggaggggc ttccatggct ggatagggcg tcttttggcg tgatgtgttc tgagttttaa 1200
agctggggaa gagactcaaa tcttcaagag ctcttccaac tttgagattc tctgttggtt 1260
tcaggactat gggaggaagc gcttgtggtc cgtgtctgtt cccgggattt ctgtttcttg 1320
gtttgtatct ctgctagagg tccaaggagc tggggcaata ccttgagtct gggttattcg 1380
tccccaggga cctgggggag cccccccccc ccacacccac acccaccccc acccccaccc 1440
ccatccccac gccccgcccc cgcccccgcc ccaaggctca ggtaaagggg agagcaaagt 1500
gtggagtttg ttccctctag tggtcagtat tagcactgca tccaggcggg cctgtctcag 1560
acgagcccag gaggagtcag caaaagtgga attcagcact gaatcatgcc cagaaccccc 1620
gcaatctatt ggctgtgctt tggccccttt tcccaacaca cacattctgt ctggtgggtg 1680
gaggggaaac atgcggggag gaggaaagga ataggataga gagtgggatg gggtcggtag 1740
gggtctcaag gactggctat cctgacatcc ttctccgcgt tcaggttggc caccatggcc 1800
tgctgccaga gggcacccac gtgaccctta aagagaggac aagttgggtg gtatctctgc 1860
tgacattctg tgcacaaccc tcacaacgct ggtgatggtg ggaagggaaa gatgacaagt 1920
cagggggcat gatcccagca tgtgtgggag gagcttctaa attatccatt agcacaagcc 1980
cgtcagtggc cccaggccta aacatgcaga gaaacaggtg aggagaagca gcgagagaga 2040
aggggccagg tataaaaagg gcccacaaga gaccagctca aggatcccaa ggcccaactc 2100
cccgaaccac tcagggtcct gtggacagct cacctagcgg caatggctgc aggtaagcgc 2160

ccctaaaatc cctttgggca caatgtgtcc tgaggggaga ggcggcgtcc tgtagatggg 2220
acgggggcac taaccctcag gtttggggct tatgaatgtt agtatcgcca tctaagccca 2280
gtatttggcc aatctctgaa tgttcctggt ccctggaggg aggcagagag agagagagag 2340
aaaaaaaaaa cccagctcct ggaacaggga gagcgctggc ctcttgctct ccagctccct 2400
ctgttgcctc cggtttctcc ccaggctccc ggacgtccct gctcctggct tttggcctgc 2460
tctgactgtc ctggcttcaa gagggcagtg ccttcccaac cattccctta tccaggcttt 2520
ttgacaacgc tatgctccgc gcccgtcgcc tgtaccagct ggcatatgac acctatcagg 2580
agtttgtaag ctcttgggta atgggtgcgc ttcagaggtg gcaggaaggg gtgaatttcc 2640
cccgctggga agtaatggga ggagactaag gagctcaggg ttgttttctg aagtgaaaat 2700
gcaggcagat gagcatacgc tgagtgaggt tcccagaaaa gtaacaatgg gagctggtct 2760
ccagcataga ccttggtggg cggtccttct cctaggaaga agcctatatc ctgaaggagc 2820
agaagtattc attcctgcag aacccccaga cctccctctg cttctcagag tctattccaa 2880
caccttccaa cagggtgaaa acgcagcaga aatctgtgag tggatgcctt ctccccaggt 2940
gggatggggt agacctgtgg tcagagcccc cgggcagcac agccactgcc ggtccttccc 3000
ctgcagaacc tagagctgct ccgcatctcc ctgctgctca tccagtcatg gctggagccc 3060
gtgcagctcc tcaggagcgt cttcgccaac agcctggtgt atggcgcctc ggacagcaac 3120
gtctatcgcc acctgaagga cctagaggaa ggcatccaaa cgctgatgtg ggtgagggtg 3180
gcaccaggga tccccaatcc tggggcccca ctggcttcca gggactgggg agagaaacac 3240
tgctgccctc tttttagcag tcaggcgctg acccaagaga actcaccgta ttcttcattt 3300
cccctcgtga atcctccagg cctttctcta caacctggag ggagggaggg aaaatggatg 3360
aatgagagag ggagggaaca gtgcccaagc gcttggcctc tccttctctt ccttcacttt 3420
gcagaggctg gaagatggca gcccccggac tgggcagatc ttcaatcagt cctacagcaa 3480
gtttgacaca aaatcgcaca acgatgacgc actgctcaag aactacgggc tgctctactg 3540
cttcaggaag gacatggaca aggtcgagac attcctgcgc atcgtgcagt gccgctctgt 3600
ggagggcagc tgtggcttct agctgcccgg gtggcatccc tgtgacccct ccccagtgcc 3660
tctcctggtc gtggaaggtg ctactccagt gcccaccagc cttgtcctaa taaaattaag 3720
ttgcatcatt ttgtttgact aggtgtcctt gtataatatt atggggtgga ggcgggtggt 3780
atggagcaag gggcaggttg ggaagacaac ctgtagggcc ttcagggtct attgggaacc 3840
aggctggagt gcagtggcac gatcttggct cgctgcaatc tccgcctcct gggttcaagc 3900
gattctcctg cctcagtctc ccgaatagtt gggattccag gcatgcacga ccaggctcag 3960
ctaatttttg tatttttggt agagacgggg tttcaccata ttggccagtc tggtctccat 4020
ctcctgacct caggtaatcc gcccgcctcg gcctcccaaa ttgctgggat tacaggtatg 4080
agccactggg cccttccctg tcctgtgatt ttaaaataat tataccagca gaaggacgtc 4140
cagacacagc atgggctacc tggccatgcc cagccagttg gacatttgag ttgtttgctt 4200
ggcactgtcc tctcatgcat tgggtccact cagtagatgc ttgttgaatt cctgggcctg 4260
gggctgtgtc agctgcctcg tcccatcacc ttctggctta ttctctccct ccgtatctta 4320
gctttttact tcatgggaat gtttccaatt cgaaatttct attttaccgt tatatattta 4380
cttttttgct gttttctcgg cccccagtag attgttagct ccagaagaga aaggatcatg 4440
tcttttgttt atctagatat gcccatctgc ctggtacaat ctctggcaca tgttataggc 4500
aacaactact tgtggaattg gtgaatgcac gaatagaaga atgagtgaat gaatgaatag 4560
acgaaaggca gaattccagc ctcaggcagg gcgcggtggc tcacgcctgt aatcccagca 4620
ctttgggagg tcgaggcagg tggatcacga ggtcaggagt tcgagaccat cctggctaac 4680
acagtgaaac cccacctcta aaaatacaaa aaaattagct gggcaaggtg gcgggcgcct 4740
gtactcccag ctactcggga ggctgaggca ggagaatgtc atgaacccca gggggcagag 4800
cttgcagtga gccgagatcg cgccactgca ctccagcctg ggtgacagag caagactcgg 4860
tctcaaaaaa aaagaaagaa agaaatccag cctcaaagag cttacagtct ggtaagggga 4920
ataaaaatgt ctgcaaatag ccacaggaca ggtcaaagga aggagaggct atttccagct 4980
gagggcaccc catcaggaaa gc 5002

<210> 2
<211> 654
<212> ADN
<213> Homo sapiens

<400> 2
atggctgcag gctcccggac gtccctgctc ctggcttttg gcctgctctg actgtcctgg 60

cttcaagagg gcagtgcctt cccaaccatt cccttatcca ggctttttga caacgctatg 120
ctccgcgccc gtcgcctgta ccagctggca tatgacacct atcaggagtt tgaagaagcc 180
tatatcctga aggagcagaa gtattcattc ctgcagaacc cccagacctc cctctgcttc 240
tcagagtcta ttccaacacc ttccaacagg gtgaaaacgc agcagaaatc taacctagag 300
ctgctccgca tctccctgct gctcatccag tcatggctgg agcccgtgca gctcctcagg 360
agcgtcttcg ccaacagcct ggtgtatggc gcctcggaca gcaacgtcta tcgccacctg 420
aaggacctag aggaaggcat ccaaacgctg atgtggaggc tggaagatgg cagcccccgg 480
actgggcaga tcttcaatca gtcctacagc aagtttgaca caaaatcgca caacgatgac 540
gcactgctca agaactacgg gctgctctac tgcttcagga aggacatgga caaggtcgag 600
acattcctgc gcatcgtgca gtgccgctct gtggagggca gctgtggctt ctag 654

<210> 3
<211> 771
<212> ADN
<213> Homo sapiens

<400> 3
atggctgcag gctcccggac gtccctgctc ctggcttttg gcctgctctg actgtcctgg 60
cttcaagagg gcagtgcctt cccaaccatt cccttatcca ggctttttga caacgctatg 120
ctccgcgccc gtcgcctgta ccagctggca tatgacacct atcaggagtt tgaagaagcc 180
tatatcctga aggagcagaa gtattcattc ctgcagaacc cccagacctc cctctgcttc 240
tcagagtcta ttccaacacc ttccaacagg gtgaaaacgc agcagaaatc taacctagag 300
ctgctccgca tctccctgct gctcatccag tcatggctgg agcccgtgca gctcctcagg 360
agcgtcttcg ccaacagcct ggtgtatggc gcctcggaca gcaacgtcta tcgccacctg 420
aaggacctag aggaaggcat ccaaacgctg atgtgggtga gggtggcacc agggatcccc 480
aatcctgggg ccccactggc ttccagggac tggggagaga aacactgctg ccctcttttt 540
agcagtcagg cgctgaccca agagaactca ccgtattctt catttcccct cgtgaatcct 600
ccaggccttt ctctacaacc tggaggggag ggaggaaaat ggatgaatga gagagggagg 660
gaacagtgcc caagcgcttg gcctctcctt ctcttccttc actttgcaga ggctggaaga 720
tggcagcccc cggactgggc agatcttcaa tcagtcctac agcaagtttg a 771

<210> 4
<211> 738
<212> ADN
<213> Homo sapiens

<400> 4
atggctgcag gctcccggac gtccctgctc ctggcttttg gcctgctctg actgtcctgg 60
cttcaagagg gcagtgcctt cccaaccatt cccttatcca ggctttttga caacgctatg 120
ctccgcgccc gtcgcctgta ccagctggca tatgacacct atcaggagtt tgaagaagcc 180
tatatcctga aggagcagaa gtattcattc ctgcagaacc cccagacctc cctctgcttc 240
tcagagtcta ttccaacacc ttccaacagg gtgaaaacgc agcagaaatc taacctagag 300
ctgctccgca tctccctgct gctcatccag tcatggctgg agcccgtgca gctcctcagg 360
agcgtcttcg ccaacagcct ggtgtatggc gcctcggaca gcaacgtcta tcgccacctg 420
aaggacctag aggaaggcat ccaaacgctg ataggctgga agatggcagc ccccggactg 480
ggcagatctt caatcagtcc tacagcaagt ttgacacaaa atcgcacaac gatgacgcac 540
tgctcaagaa ctacgggctg ctctactgct tcaggaagga catggacaag gtcgagacat 600
tcctgcgcat cgtgcagtgc cgctctgtgg agggcagctg tggcttctag ctgcccgggt 660
ggcatccctg tgacccctcc ccagtgcctc tcctggtcgt ggaaggtgct actccagtgc 720
ccaccagcct tgtcctaa 738

<210> 5
<211> 609
<212> ADN

<213> Homo sapiens

<400> 5
atggctgcag gctcccggac gtccctgctc ctggcttttg gcctgctctg actgtcctgg 60
cttcaagagg gcagtgcctt cccaaccatt cccttatcca ggctttttga caacgctatg 120
ctccgcgccc gtcgcctgta ccagctggca tatgacacct atcaggagtt taacccccag 180
acctccctct gcttctcaga gtctattcca acaccttcca acagggtgaa aacgcagcag 240
aaatctaacc tagagctgct ccgcatctcc ctgctgctca tccagtcatg gctggagccc 300
gtgcagctcc tcaggagcgt cttcgccaac agcctggtgt atggcgcctc ggacagcaac 360
gtctatcgcc acctgaagga cctagaggaa ggcatccaaa cgctgatgtg gaggctggaa 420
gatggcagcc cccggactgg gcagatcttc aatcagtcct acagcaagtt tgacacaaaa 480
tcgcacaacg atgacgcact gctcaagaac tacgggctgc tctactgctt caggaaggac 540
atggacaagg tcgagacatt cctgcgcatc gtgcagtgcc gctctgtgga gggcagctgt 600
ggcttctag 609

<210> 6
<211> 51
<212> ADN
<213> Homo sapiens

<400> 6
atggctgcag gctcccggac gtccctgctc ctggcttttg gcctgctctg a 51

<210> 7
<211> 16
<212> PRT
<213> Homo sapiens

<400> 7
Met Ala Ala Gly Ser Arg Thr Ser Leu Leu Leu Ala Phe Gly Leu Leu
1               5                   10                  15

<210> 8
<211> 18
<212> ADN
<213> Homo sapiens

<400> 8
gtgttggcct cttgctct 18

<210> 9
<211> 18
<212> ADN
<213> Homo sapiens

<400> 9
ggaaagtcac cccttcct 18

## Description

The present invention relates to new polynucleotides comprising a SNP in the nucleotide sequence of the hGH-V gene, new polypeptides comprising a mutation caused by this SNP as well as their therapeutic uses.

### PRIOR ART

The human placental growth hormone gene, hereinafter referred to as hGH-V, is described in the publications :
- George,D.L., Phillips,J.A. III, Francke,U. and Seeburg,P.H. (1981). The genes for growth hormone and chorionic somatomammotropin are on the long arm of human chromosome 17 in region q21 to qter. Hum. Genet. 57 (2), 138-141.
- Harper,M.E., Barrera-Saldana,H.A. and Saunders,G.F. (1982). Chromosomal localization of the human placental lactogen-growth hormone gene cluster to 17q22-24. Am. J. Hum. Genet. 34 (2), 227-234.
- Kidd,V.J. and Saunders,G.F. (1982). Linkage arrangement of human placental lactogen and growth hormone genes. J. Biol. Chem. 257 (18), 10673-10680.
- Barsh,G.S., Seeburg,P.H. and Gelinas,R.E. (1983). The human growth hormone gene family: structure and evolution of the chromosomal locus. Nucleic Acids Res. 11 (12), 3939-3958.
- lgout,A., Scippo,M.L., Frankenne,F. and Hennen,G. (1988). Cloning and nucleotide sequence of placental hGH-V cDNA. Arch. Int. Physiol. Biochim. 96 (1), 63-67.
- Cooke,N.E., Ray,J., Emery,J.G. and Liebhaber,S.A. (1988). Two distinct species of human growth hormone-variant mRNA in the human placenta predict the expression of novel growth hormone proteins. J. Biol. Chem. 263 (18), 9001-9006.
- Chen,E.Y., Liao,Y.C., Smith,D.H., Barrera-Saldana,H.A., Gelinas,R.E. and Seeburg,P.H. The human growth hormone locus. Genomics 4 (4), 479-497.
- Vnencak-Jones,C.L. and Phillips,J.A. III. (1990). Hot spots for growth hormone gene deletions in homologous regions outside of Alu repeats. Science 250 (4988), 1745-1748.
- MacLeod,J.N., Lee,A.K., Liebhaber,S.A. and Cooke,N.E. (1992). Developmental control and alternative splicing of the placentally expressed transcripts from the human growth hormone gene cluster. J. Biol. Chem. 267 (20), 14219-14226.
- Boguszewski,C.L., Svensson,P.A., Jansson,T., Clark, R., Carlsson,L.M. and Carlsson,B. (1998). Cloning of two novel growth hormone transcripts expressed in human placenta. J. Clin. Endocrinol. Metab. 83 (8), 2878-2885.

The nucleotide sequence of this gene is accessible under accession number JO3071 in the GenBank database.

Placental growth hormone (PGH) is the product of the hGH-V gene.

The human growth hormone (hGH)/human placental lactogen (hPL) gene family, which consists of two hGH (pituitary GH and placental GH) and three hPL genes, is involved in the regulation of maternal and fetal metabolism and the growth and development of the fetus.

During pregnancy, pituitary GH (hGH-N) expression in the mother is suppressed and hGH-V, that is a GH variant expressed by the cells from the syncytiotrophoblast layer of the human placenta, becomes the predominant GH in the mother. Assays of PGH by specific monoclonal antibodies reveal that, in the maternal circulation from 15-20 weeks up to term, PGH gradually replaces pituitary growth hormone which becomes undetectable (Alsat et al. (1998); Physiological role of human placental growth hormone. Mol. Cell Endocrinol. 140 :121-127).

hGH-N, which is expressed by the fetal pituitary, has little or no physiological actions in the fetus until late in pregnancy due to the lack of functional GH receptors on fetal tissues (Handwerger et al. (2000); The roles of placental growth hormone and placental lactogen in the regulation of human fetal growth and development; J. Pediatr Endocrinol. Metab. 13:343-356).

By contrast, PGH is continuously secreted by the placenta. It has high somatogenic and low lactogenic activities. PGH secretion appears to have important implications for physiological adjustment to gestation and especially in the control of maternal IGF1 levels. Thus, it acts as a growth-promoting hormone and appears to be the main stimulator of insulin-like growth factor I (IGF-I) secretion (Caron et al. (1997); Expression of somatostatin receptor SST4 in human placenta and absence of octreotide effect on human placental growth hormone concentration during pregnancy. J. Clin. Endocrinol. Metab. 82:3771-3776).

Moreover, PGH may continue to have an effect on the small child after birth, as suggested by the Karlberg's model, which corresponds to a model differentiating between three discrete, but related periods of postnatal growth : fetal/infant, childhood and pubertal phases (Karlberg J. (1990); The infancy-childhood growth spurt. Acta. Paediatr. Scand. Suppl. 367:111-118). During these periods, growth is regulated by different hormonal control systems. In particular, the infant period is commonly seen as a continuation of fetal life and the insulin like growth factor system is proposed to be the most important factor regulating growth. Growth in childhood is affected by both environmental and genetic influences.

PGH may not only act on fetal or young child growth. Indeed, it has been shown by reverse transcriptase-polymerase chain reaction (RT-PCR) analysis that hGH-N and hGH-V transcripts are simultaneously produced by human peripheral blood mononuclear cells in both men and women as well as pregnant women. Thus, hGH-V mRNA is expressed by cells other than the syncytiotrophoblast, is not regulated by PIT-1 (pituitary transcription factor 1), and may be involved in immune regulation, as is pituitary GH (Melen et al. (1997). Both pituitary and placental growth hormone transcripts are expressed in human peripheral blood mononuclear cells (PBMC) (Clin. Exp. Immunol. 110:336-340).

The biological actions of hGH-V are mediated through binding to specific, high affinity receptors localized to the plasma membrane of target tissues. The affinity of hGH-V for binding to the GH receptor is identical to that of hGH-N. The expression of GH receptor mRNA in human fetal tissues has been examined using the technique of reverse transcription-polymerase chain reaction (RT-PCR). Messenger RNA encoding the membrane-bound GH receptor is expressed in the liver, kidney, skin, muscle, lung, adrenal, brain, spleen, intestine and pancreas of the human fetus at 7-20 weeks of gestation. Immunohistochemistry analysis indicates a progressive maturation of GH receptor in late gestation or in the perinatal period (Handwerger and Freemark (2000); The roles of placental growth hormone and placental lactogen in the regulation of human fetal growth and development. J. Pediatr. Endocrinol. Metab. 13:343-356). As a consequence, growth hormone may exert growth-promoting and metabolic effects on many different tissues.

Information concerning the physiological role of human placental growth hormone can be found in additional articles and reviews like :
- Alsat et al. (1997). Human placental growth hormone. Am. J. Obstet. Gynecol. 177:1526-1534.
- Chappel and Murphy (2000). Growth hormone. In : Cytokine reference, Academic Press. 251-265.

Human growth hormone modulates the production of IGF-1 in the pregnant mother, and plays important roles in perinatal carbohydrate metabolism, phallic growth and craniofacial developments.

The physiological activity for which human adult GH is best known is the promotion of growth of bone, cartilage and soft tissue. Adult GH appears also to control important immune functions. GH has been shown to be produced by T-cells, B-cells and macrophages. Receptors for GH releasing hormone have also been found on cells of the immune system. GH appears to act as an enhancer of immune responses and is produced in considerable amounts by T-helper cells.

Hypothalamus-derived GH releasing hormone has been shown recently to elicit GH production by lymphocytes. GH augments the cytolytic activity of T-cells, antibody synthesis, and granulocyte differentiation induced by GM-CSF. GH also enhances production of TNF-alpha, generation of superoxide anions from peritoneal macrophages, and natural killer activity. GH induces a chemotactic response in human monocytes which is inhibited by Somatostatin. GH enhances the synthesis of some Thymic hormones. An active fragment of GH appears to account for some of the biological activity of PM (pregnancy mitogen ). GH has been shown to promote engraftment of murine or human T-cells in severe combined immunodeficient mice (SCID) mice.

Treatment of mice with recombinant human GH has been shown to partially counteract the myelosuppressive properties of azidothymidine, resulting in an increase in splenic hematopoietic progenitor cells. GH has been shown to function as a paracrine mediator of growth and differentiation in the hematopoietic system.

A recently reported action of GH is its support of angiogenesis.

Many recent studies on adults lacking growth hormone demonstrated that growth hormone exerts an effect on lipid metabolism and prevents arteriosclerosis.

PGH secretion seems to have important implications in the control of maternal IGF1 levels.

Deficiency of IGF1 was proposed as the nature of the basic defect in the African pygmy and possibly also in the Laron type of dwarfism.

IGF1 gene may be the site of the mutation causing one form of hypochondroplasia.

IGF1 stimulates skeletal muscle hypertrophy and a switch to glycolytic metabolism by activating the calcium calmodulin-dependent phosphatase calcineurin and inducing the nuclear translocation of transcription factor NFATC1.

A strong association between circulating IGF1 concentrations and the risk of breast cancer in premenopausal has been demonstrated.. Holly discussed the evidence that high levels of circulating IGF1 pose a risk of breast cancer in premenopausal women, and noted that a similar association has been reported for prostate cancer (Holly (1998). Insulin-like growth factor-I and new opportunities for cancer prevention. Lancet 351: 1373-1375).

Circulating IGF1 may play a role in the age-related reduction of certain cognitive functions, specifically speed of information processing (Aleman et al. (1999). Insulin-like growth factor-I and cognitive function in healthy older men. J. Clin. Endocr. Metab. 84: 471-475).

IGF-1 may contribute to transformation, cell migration, and a propensity for metastasis in vivo (Playford et al. (2000). Insulin-like growth factor 1 regulates the location, stability, and transcriptional activity of beta-catenin. Proc. Nat. Acad. Sci. 97: 12103-12108).

A 15-year-old boy with severe prenatal and postnatal growth failure, sensorineural deafness, and mental retardation who was homozygous for a partial deletion of the IGF1 gene has been described (Woods et al. (1996). Intrauterine growth retardation and postnatal growth failure associated with deletion of the insulin-like growth factor I gene. New Eng. J. Med. 335: 1363-1367).

Rasmussen et al. considered the IGF1 and IGF1R genes as candidates for low birth weight, insulin resistance, and type II diabete (Rasmussen et al. (2000). Studies of the variability of the genes encoding the insulin-like growth factor I receptor and its ligand in relation to type 2 diabetes mellitus. J. Clin. Endocr. Metab. 85: 1606-1610).

As indicated by experiments on knock-out mouse, IGF1 may have specific roles in axonal growth and myelination. In addition, neonatal mortality is substantial, suggesting that the defect may be lethal in humans also.

Hellstrom et al. showed that lack of IGF1 in knockout mice prevents normal retinal vascular growth, despite the presence of vascular endothelial growth factor, which is important for vessel development (Hellstrom et al. (2001). Low IGF-I suppresses VEGF-survival signaling in retinal endothelial cells: direct correlation with clinical retinopathy of prematurity. Proc. Nat. Acad. Sci. 98: 5804-5808).

The nucleotide sequence of the wild hGH-V gene of reference, mentioned in the GenBank accession number JO3071, comprises 5001 nucleotides.

However, it appears that this nucleotide sequence is not exact. The inventor has noted that the nucleotide coding sequence of the variant N°2 of the hGH-V gene reported in the GenBank database (accession number : NM_022557) comprises an additional cytosine (c) at position 480. This encodes for the amino acid sequence of PGH variant N°2 as shown by SwissProt database N° P09587.

By contrast, the nucleotide sequence of hGH-V gene deposited in the Genbank database (accession number: J03071) as such does not comprise a cytosine (c) between position 3194 and 3195. Such a nucleotide sequence would be responsible for an altered PGH variant N°2 due to the apparition of a frameshift, which would be different from PGH variant N°2 deposited in the SwissProt database N° P09587.

Consequently, to be in agreement with the coding sequence of the variant N°2 of the hGH-V gene reported in the GenBank database (accession number : NM_022557), the inventor has considered that a correction was necessary. This correction consists in adding a cytosine (c) between positions 3194 and 3195 in the nucleotide sequence of hGH-V gene deposited in the GenBank database (accession number: J03071). This corrected sequence is named hereinafter "corrected nucleotide sequence of wild hGH-V gene".

The nucleotide sequence SEQ ID N°1 contains 5 exons (as defined by the splicing sites, which generate the normal variant N°1 of the PGH) as follows:
- Exon 1 : nucleotide 2143 to 2152;
- Exon 2 : nucleotide 2425 to 2585;
- Exon 3 : nucleotide 2796 to 2915;
- Exon 4 : nucleotide 3007 to 3171; and
- Exon 5 : nucleotide 3425 to 3622.

The predominant protein 22-kDa isoform encoded by hGH-V is composed of 217 amino acids and utilizes all five exons of the corrected nucleotide sequence of the wild hGH-V gene.

This isoform, hereinafter referred to as variant N°1, is encoded by a coding nucleotide sequence composed of 654 nucleotides. The 217 amino acid sequence corresponds to an immature protein, that will be converted to a mature protein of 191 amino acids, by cleavage of the signal peptide that includes the 26 first amino acids. The nucleotide coding sequence of the variant N°1 is accessible under accession number NM_002059 in the GenBank database.

Variant N°2 utilizes intron situated between introns 4 and 5 to generate the longest isoform of 256 amino acids, which diverges from all other GH isoforms in the carboxy terminus. This isoform is encoded by a coding nucleotide sequence composed of 771 nucleotides corresponding to nucleotides 2143 to 2152; nucleotides 2425 to 2585, nucleotides 2796 to 2915 and nucleotides 3007 to 3486 of the corrected nucleotide sequence of wild hGH-V gene. The 256 amino acid sequence corresponds to an immature protein, that will be converted to a mature protein of 230 amino acids, by cleavage of the signal peptide that includes the 26 first amino acids. The nucleotide coding sequence of the variant N°2 is accessible under accession number NM_022557 in the GenBank database.

In addition to the variant N°1 and N°2 mentioned above, two other variants are known.

Variant N°3 utilizes an alternative splice donor site in exon 4 causing a 4 nucleotide deletion and a frameshift which generates an isoform of 245 amino acids with an unique carboxy-terminus. This isoform is encoded by a coding nucleotide sequence composed of 738 nucleotides corresponding to nucleotides 2143 to 2152; nucleotides 2425 to 2585, nucleotides 2796 to 2915, nucleotides 3007 to 3167 and nucleotide 3425 to 3710 of the corrected nucleotide sequence of wild hGH-V gene. The 245 amino acid sequence corresponds to an immature protein, that will be converted to a mature protein of 219 amino acids, by cleavage of the signal peptide that includes the 26 first amino acids. The nucleotide coding sequence of the variant N°3 is accessible under accession number NM_022558 in the GenBank database.

Variant N°4 utilizes an alternative splice acceptor site of 45 nucleotides into exon 3 to generate the 20-kDa isoform composed of 202 amino acids, which has an internal deletion relative to the predominant 22-kDa isoform (variant N°1). This isoform is encoded by a coding nucleotide sequence composed of 609 nucleotides corresponding to nucleotides 2143 to 2152; nucleotides 2425 to 2585, nucleotides 2841 to 2915, nucleotides 3007 to 3171 and nucleotide 3425 to 3622 of the corrected nucleotide sequence of the wild hGH-V gene. The 202 amino acid sequence corresponds to an immature protein, that will be converted to a mature protein of 176 amino acids, by cleavage of the signal peptide that includes the 26 first amino acids. The nucleotide coding sequence of the variant N°4 is accessible under accession number NM_022556 in the GenBank database.

### THE INVENTION

The applicant has identified one SNP in the corrected nucleotide sequence of the wild hGH-V gene.

The first object of the invention concerns new polynucleotides defined as the corrected nucleotide sequence and differing from the wild hGH-V gene in that it comprises the new SNP (Single Nucleotide Polymorphism) : c2465a.

The nucleotide sequence SEQ ID N°1 corresponds to the corrected nucleotide sequence of the wild hGH-V gene mentioned above comprising 5002 nucleotides, containing the SNP c2465a.

This SNP corresponds to the substitution of a cytosine (c) by an adenine (a) at position 2465 of the corrected nucleotide sequence of the wild hGH-V gene. This SNP hereinafter is indifferently designated as c2465a and c51a. The SNP c51a appears in each of the nucleotide coding sequence of variants N°1, 2, 3 and 4 at their respective position 51.

The nucleotide coding sequence SEQ ID N°2 corresponds to the nucleotide coding sequence of the variant N°1 of the hGH-V gene containing the SNP c51a.

The nucleotide coding sequence SEQ ID N°3 corresponds to the nucleotide coding sequence of the variant N°2 containing the SNP c51a.

The nucleotide coding sequence SEQ ID N°4 corresponds to the nucleotide coding sequence of the variant N°3 of the hGH-V gene containing the SNP c51a.

The nucleotide coding sequence SEQ ID N°5 corresponds to the nucleotide coding sequence of the variant N°4 of the hGH-V gene containing the SNP c51a.

This SNP has been identified by the applicant using the determination process described in applicant's patent application FR 00 22894, entitled "Process for the determination of one or several functional polymorphism(s) in the nucleotide sequence of a preselected functional candidate gene and its applications" and filed December 6, 2000, cited here by way of reference.

The process described in this patent application permits the identification of one (or several) preexisting SNPs in at least one individual from a random population of individuals.

In the scope of the present invention, a fragment of the nucleotide sequence of the hGH-V gene, comprising, for example, the coding sequence, was isolated from among individuals in a population of individuals chosen in a random manner.

Sequencing of these fragments was then carried out on certain of these samples having a heteroduplex profile (that is a profile different from that of the reference wild nucleotide sequence of hGH-V gene) after analysis by DHPLC ("Denaturing-High Performance Liquid Chromatography").

The fragment sequenced in this way was then compared to the nucleotide sequence of the fragment of the reference wild hGH-V gene and the SNP in conformity with the invention identified.

Thus the SNP is natural and present in certain individuals of the world population more particularly in the south American population.

The SNP c2465a (or c51a) involves a modification of the PGH protein encoded by the nucleotide sequence of the hGH-V gene at the level of the amino acid sequence. This SNP introduces a stop codon "tga" at positions 2463-2465 on the nucleotide sequence of the hGH-V gene and at positions 49-51 on the nucleotide coding sequence of variants N°1 to 4 of the hGH-V gene.

As a consequence, this SNP is responsible for the formation of a stop codon during the translation process at position 17 of the immature protein of PGH. The resulting amino acids sequence corresponds to a fragment of the sixteen first amino acids of the signal peptide.

The amino acids sequence SEQ ID N°7 corresponds to the sixteen first amino acids of the immature protein of PGH.

The nucleotide sequence SEQ ID N°6 corresponds to the nucleotide coding sequence deduced from the hGH-V gene encoding for the amino acids sequence SEQ ID N°7. The nucleotide coding sequence SEQ ID N°6 contains an adenine (a) at position 51.

Genotyping of the polynucleotides in conformity with the invention containing the SNP of the invention can be carried out in such a way as to determine the allelic frequency of these polynucleotides in a population.

The invention also has for an object the use of polynucleotides and of polypeptides of the invention as well as of therapeutic molecules obtained and/or identified starting from these polynucleotides and polypeptides, notably for the prevention and the treatment of certain human disorders and/or diseases.

### DETAILED DESCRIPTION OF THE INVENTION

### Definitions

By "natural PGH" is understood the mature placental growth hormone (PGH) encoded by the nucleotide sequence of the reference wild hGH-V gene.

By "polynucleotide" is understood a polyribonucleotide or a polydeoxyribonucleotide that can be a modified or a non-modified DNA or an RNA.

The term polynucleotide includes, for example, a single strand or double strand DNA, a DNA composed of a mixture of one or several single strand region(s) and of one or several double strand region(s), a single strand or double stand RNA and an RNA composed of a mixture of one or several single strand region(s) and of one or several double strand region(s). The term polynucleotide can also include an RNA and/or a DNA including one or several triple strand regions. By polynucleotide is equally understood the DNAs and RNAs containing one or several bases modified in such a fashion as to have a skeleton modified for reasons of stability or for other reasons. By modified base is understood, for example, the unusual bases such as inosine.

By "polypeptide" is understood a peptide, an oligopeptide, an oligomer or a protein comprising at least two amino acids joined to each other by a normal or modified peptide bond, such as in the cases of the isosteric peptides, for example.

A polypeptide can be composed of amino acids other than the 20 amino acids encoded by human genes. A polypeptide can equally be composed of amino acids modified by natural processes, such as post translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural or synthetic post-translational processes that are well known to a person skilled in the art.

For example, by polypeptide modifications is understood acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bridge formation, demethylation, cysteine formation, pyroglutamate formation, formylation, gamma-carboxylation, glycosylation, GPI anchor formation, hydroxylation, iodization, methylation, myristoylation, oxidation, proteolytic processes, phosphorylation, prenylation, racemization, la seneloylation, sulfatation, amino acid addition such as arginylation or ubiquitination. Such modifications are fully detailed in the literature: PROTEINS-STRUCTURE AND MOLECULAR PROPERTIES, 2^{nd} Ed., T. E. Creighton, New York, 1993, POST-TRANSLATIONAL COVALENT MODIFICATION OF PROTEINS, B. C. Johnson, Ed., Academic Press, New York, 1983, Seifter et al. "Analysis for protein modifications and nonprotein cofactors", Meth. Enzymol. (1990) 182:626-646 et Rattan et al. "Protein Synthesis: Post-translational Modifications and Aging", Ann NY Acad Sci (1992) 663: 48-62.

By "isolated polynucleotide" or "isolated polypeptide" is understood a polynucleotide or a polypeptide such as previously defined which is isolated from the human body or otherwise produced by a technical process.

By "identity" is understood the measurement of nucleotide or polypeptide sequence identity. Identity is a term well known to a person skilled in the art and in the literature. See COMPUTATIONAL MOLECULAR BIOLOGY, Lesk, A.M., Ed., Oxford University Press, New York, 1998; BIOCOMPUTING INFORMATICS AND GENOME PROJECT, Smith, D.W., Ed., Academic Press, New York, 1993; COMPUTER ANALYSIS OF SEQUENCE DATA, PART I, Griffin, A.M. and Griffin H.G., Ed, Humana Press, New Jersey, 1994; et SEQUENCE ANALYSIS IN MOLECULAR BIOLOGY, von Heinje, G., Academic Press, 1987.

The methods commonly employed to determine the identity and the similarity between two sequences are equally well described in the literature. See GUIDE TO HUGE COMPUTER, Martin J. Bishop, Ed, Academic Press, San Diego, 1994, et Carillo H. and Lipton D., Siam J Applied Math (1988) 48: 1073.

A polynucleotide having, for example, an identity of at least 95 % with the nucleotide sequence SEQ ID N° 1 is a polynucleotide which contains at most 5 points of mutation over 100 nucleotides, compared to said sequence.

These points of mutation can be one (or several) substitution(s), addition(s) and/or deletion(s) of one (or several) nucleotide(s).

In the same way, a polypeptide having, for example, an identity of at least 95 % with the amino acid sequence SEQ ID N° 7 is a polypeptide that contains at most 5 points of mutation over 100 amino acids, compared to said sequence.

These points of mutation can be one (or several) substitution(s), addition(s) and/or deletion(s) of one (or several) amino acid(s).

The polynucleotides and the polypeptides according to the invention which are not totally identical with respectively the nucleotide sequence SEQ ID N°1 or the amino acid sequence SEQ ID N°6, it being understood that these sequences contain at least the SNP of the invention, are considered as variants of these sequences.

A variant, according to the invention, can be obtained, for example, by directed mutagenesis or by direct synthesis.

By "SNP (Single Nucleotide Polymorphism)" is understood any natural variation of a base in a nucleotide sequence. A SNP, on a nucleotide sequence, can be coding, silent or non-coding.

A coding SNP is a polymorphism included in the coding sequence of a nucleotide sequence that involves a modification of an amino acid in the sequence of amino acids encoded by this nucleotide sequence. In this case, the term SNP applies equally, by extension, to a mutation in an amino acid sequence.

A silent SNP is a polymorphism included in the coding sequence of a nucleotide sequence that does not involve a modification of an amino acid in the amino acid sequence encoded by this nucleotide sequence.

A non-coding SNP is a polymorphism included in the non-coding sequence of a nucleotide sequence. This polymorphism can notably be found in an intron, a splicing zone, a transcription promoter or a site enhancer sequence.

### Polynucleotide

The present invention has for its first object an isolated polynucleotide comprising :
a) a nucleotide sequence having at least 95 % identity, preferably at least 97 % identity, more preferably at least 99 % identity with one of the nucleotide sequences chosen in the group consisting of SEQ ID N° 1, SEQ ID N° 2, SEQ ID N° 3, SEQ ID N° 4, SEQ ID N° 5 and SEQ ID N° 6, or
b) a nucleotide sequence complementary to a nucleotide sequence under a).

It is understood that these nucleotide sequences always comprise the SNP of the invention : c2465a (or c51a).

The present invention relates equally to an isolated polynucleotide comprising :
a) one of the nucleotide sequences chosen in the group consisting of SEQ ID N° 1, SEQ ID N° 2, SEQ ID N° 3, SEQ ID N° 4, SEQ ID N° 5 and SEQ ID N° 6, or
b) a nucleotide sequence complementary to a nucleotide sequence under a).

Preferably, the polynucleotide of the invention consists of the nucleotide sequence SEQ ID N° 1 and/or of the nucleotide coding sequence SEQ ID N° 2, SEQ ID N° 3, SEQ ID N° 4, SEQ ID N° 5 and/or SEQ ID N° 6.

The present invention also has for its object an isolated polynucleotide encoding for a polypeptide consisting of the amino acid sequence SEQ ID N° 7.

Preferably a polynucleotide according to the invention is composed of a DNA or RNA molecule.

A polynucleotide according to the invention can be obtained by standard DNA or RNA synthetic methods.

A polynucleotide according to the invention can equally be obtained by directed mutagenesis staring from all or part of the nucleotide sequence of the hGH-V gene by modifying the wild cytosine (c) nucleotide by the mutated adenine (a) nucleotide at position 2465 (or 51).

The processes of directed mutagenesis that can be implemented in this way are well known to a person skilled in the art. The publication of TA Kunkel in 1985 in "Proc. Natl. Acad. Sci. USA" 82:488 can notably be mentioned.

An isolated polynucleotide can equally include, for example, nucleotide sequences coding for pre-, pro- or pre-pro-protein amino acid sequences or marker amino acid sequences, such as hexa-histidine peptide.

A polynucleotide of the invention can equally be associated with nucleotide sequences coding for other proteins or protein fragments in order to obtain fusion proteins or other purification products.

A polynucleotide according to the invention can equally include nucleotide sequences such as the 5' and/or 3' non-coding sequences, such as, for example, transcribed or non-transcribed sequences, translated or non-translated sequences, splicing signal sequences, polyadenylated sequences, ribosome binding sequences or even sequences which stabilize mRNA.

A nucleotide sequence complementary to the nucleotide or polynucleotide sequence is defined as one that can be hybridized with this nucleotide sequence, under stringent conditions.

By "stringent hybridization conditions" is generally but not necessarily understood the chemical conditions that permit a hybridization when the nucleotide sequences have an identity of at least 95 %, preferably greater than or equal to 97 %, still more preferably greater than or equal to 99 % and most preferably equal to 100 %.

The stringent conditions can be obtained according to methods well known to a person skilled in the art and, for example, by an incubation of the polynucleotides, at 42° C, in a solution comprising 50 % formamide, 5xSSC (150 mM of NaCI, 15 mM of trisodium citrate), 50 mM of sodium phosphate (pH = 7.6), 5x Denhardt Solution, 10 % dextran sulfate and 20 µg denatured salmon sperm DNA, followed by washing the filters at 0.1x SSC, at 65° C.

Within the scope of the invention, when the stringent hybridization conditions permit hybridization of the nucleotide sequences having an identity equal to 100 %, the nucleotide sequence is considered to be strictly complementary to the nucleotide sequence under a) such as described.

It is understood within the meaning of the present invention that the nucleotide sequence complementary to a nucleotide sequence comprises at least one anti-sense SNP according to the invention. Thus, for example, if the nucleotide sequence comprises the SNP c2465a, its complementary nucleotide sequence still comprises the guanine (g) nucleotide at position 2465.

### Identification, hybridization and/or amplification of a polynucleotide comprising the SNP of the invention

The present invention also has for its object the use of all or part of a polynucleotide as defined above, to identify, hybridize and/or amplify :
- a polynucleotide consisting of one of the nucleotide sequences chosen from SEQ ID N° 1, SEQ ID N° 2, SEQ ID N° 3, SEQ ID N° 4, SEQ ID N° 5 and SEQ ID N° 6; or
- a part of a polynucleotide consisting of one of the nucleotide sequences chosen from SEQ ID N° 1, SEQ ID N° 2, SEQ ID N° 3, SEQ ID N° 4, SEQ ID N° 5 and SEQ ID N° 6, provided that said part comprises the SNP c2465a (or c51a).

The present invention equally has for its object the use of all or part of a polynucleotide of the invention as a genotyping tool in the nucleotide sequence having 90 to 100 % identity with the nucleotide sequence of a growth hormone gene, this use allows to determine whether said growth hormone gene comprises the SNP c2465a (or c51a).

The nucleotide sequence of a growth hormone gene can be the nucleotide sequence of placental growth hormone (PGH) and/or the adult growth hormone (GH).

The present invention also has for its object a process for the determination of the frequency of the SNP c2465a (or c51a) in a polynucleotide according to the invention in which an individual or a population of individuals is genotyped.

Within the meaning of the invention, genotyping is defined as a process for the determination of the genotype of an individual or of a population of individuals. Genotype consists of the alleles present at one or more specific loci.

By "population of individuals" is understood a group of determined individuals selected in random or non-random fashion. These individuals can be humans, animals, microorganisms or plants.

Usually, the group of individuals comprises at least 10 persons, preferably from 100 to 300 persons.

The individuals can be selected according to their ethnicity or according to their phenotype, notably those who are affected by the following disorders and/or diseases involving the human growth and development, such as fetal growth and development, perinatal carbohydrate metabolism, phallic growth, craniofacial developments and the Laron type of dwarfism, secretion and stimulating of IGF-I, the lipid metabolism such as arteriosclerosis, angiogenesis, cancer such as breast cancer in premenopausal and prostate cancer and the immune system.

Multiple technologies exist which can be implemented in order to genotype SNPs (see notably Kwok Pharmacogenomics, 2000, vol 1, pp 95-100. "High-throughput genotyping assay approaches"). These technologies are based on one of the four following principles: allele specific oligonucleotide hybridization, oligonucleotide elongation by dideoxynucleotides optionally in the presence of deoxynucleotides, ligation allele specific oligonucleotides or cleavage of allele specific oligonucleotides. Each one of these technologies can be coupled to a detection system such as measurement of direct or polarized fluorescence, or mass spectrometry.

Genotyping can notably be carried out by minisequencing with hot ddNTPs (2 different ddNTPs labeled by different fluorophores) and cold (2 non labeled ddNTPs), in connection with a polarized fluorescence scanner. The minisequencing protocol with reading of polarized fluorescence (Technology FP-TDI or Fluorescence Polarization Template-direct Dye-Terminator Incorporation) is well known to a person skilled in the art.

It can be carried out on a product obtained after amplification by polymerase chain reaction (PCR) of the DNA of each individual. This PCR product is selected to cover the polynucleotide genic region containing the studied SNP. After the last stage in the PCR thermocycler, the plate is then placed on a polarized fluorescence scanner for a reading of the labeled bases by using fluorophore specific excitation and emission filters. The intensity values of the labeled bases are reported on a graph.

The sense and antisense primers respectively for the PCR amplification, in the case of a SNP of the invention, can easily be selected by a person skilled in the art according to the position of the SNP according to the invention.

For example, the sense and antisense nucleotide sequences for the PCR amplification can be respectively SEQ ID N° 8 and SEQ ID N° 9.

The nucleotide sequences permit amplification of a fragment having a length of 400 nucleotides in the nucleotide sequence SEQ ID N° 1.

A statistical analysis of the frequency of each allele (allelic frequency) encoded by the gene comprising the SNP in the population of individuals is thus achieved, which permits determination of the importance of their impact and their distribution in the different sub-groups and notably, if necessary, the diverse ethnic groups that constitute this population of individuals.

The genotyping data are analyzed in order to estimate the distribution frequency of the different alleles observed in the studied populations. The calculations of the allelic frequencies can be carried out with the help of software such as SAS-suite® (SAS) or SPLUS® (MathSoft). The comparison of the allelic distributions of a SNP of the invention across different ethnic groups of the population of individuals can be carried out by means of the software ARLEQUIN® and SAS-suite®.

### Expression vector and host cell

The present invention also has for its object a recombinant vector comprising at least one polynucleotide according to the invention.

Numerous expression systems can be used, like, for example, chromosomes, episomes, derived viruses. More particularly, the recombinant vectors used can be derived from bacterial plasmids, transposons, yeast episome, insertion elements, yeast chromosome elements, viruses such as baculovirus, papilloma viruses such as V40, vaccinia viruses adenoviruses, fox pox viruses, pseudorabies viruses, retroviruses.

These recombinant vectors can equally be cosmid or phagemid derivatives. The nucleotide sequence can be inserted in the recombinant expression vector by methods well known to a person skilled in the art such as, for example, those that are described in MOLECULAR CLONING, A LABORATORY MANUAL (supra) Sambrook et al.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the polynucleotide of the invention, will be directed towards the opening of the endoplasmic reticulum, towards the periplasmic space, on the membrane or towards the extracellular environment.

The present invention also has for its object a host cell comprising a recombinant vector according to the invention.

The introduction of the recombinant vector in a host cell can be carried out according to methods that are well known to a person skilled in the art such as those described in BASIC METHODS IN MOLECULAR BIOLOGY, Davis et al., 1986 et MOLECULAR CLONING: A LABORATORY MANUAL, 2^{nd} Ed., Cold Spring Harbor Laboratory Press, Cold Spring Harbor, N.Y., 1989, such as transfection by calcium phosphate, transfection by DEAE dextran, transfection, microinjection, transfection by cationic lipids, electroporation, transduction or infection.

The host cell can be, for example, bacterial cells such as cells of streptococci, staphylococci, *E. coli* or *Bacillus subtilis,* cells de fungi such as yeast cells and cells of *Aspergillus*, *Streptomyces,* insect cells such as cells of *Drosophilia* S2 and of *Spodoptera* Sf9, animal cells, such as CHO, COS, HeLa, C127, BHK, HEK 293 cells and human cells of the subject to treat or even plant cells.

The host cells can be used, for example, to express a polypeptide of the invention or as an active product in pharmaceutical compositions, as will be seen hereinafter.

### Polypeptide

The present invention also has for its object an isolated polypeptide comprising an amino acid sequence having at least 95 % identity, preferably 97 % identity, more preferably 99 % identity, with the amino acid sequence SEQ ID N° 7.

The polypeptide of the invention can equally comprise the amino acid sequence SEQ ID N° 7.

The polypeptide of the invention can more particularly consist of the amino acid sequence SEQ ID N° 7.

The present invention equally has for its object a process for the preparation of the above-described polypeptide, in which a previously defined host cell is cultivated in a culture medium and said polypeptide is isolated from the culture medium.

The polypeptide can be purified starting from the host cells, according to methods well known to a person skilled in the art such as precipitation with the help of chaotropic agents such as salts, in particular ammonium sulfate, ethanol acetone or trichloroacetic acid, acid extraction; ion exchange chromatography; phosphocellulose chromatography; hydrophobic interaction chromatography; affinity chromatography; hydroxyapatite chromatography or exclusion chromatographies.

By "culture medium" is understood the medium in which the polypeptide of the invention is isolated or purified. This medium can be composed of the extracellular medium and/or the cellular lysate. Techniques well known to a person skilled in the art equally permit the latter to give back an active conformation to the polypeptide, if the conformation of said polypeptide was altered during the isolation or the purification.

### Antibodies

The present invention also has for its object a process for obtaining an immunospecific antibody.

By "antibody" is understood the monoclonal, polyclonal, chimeric, simple chain, humanized antibodies as well as the Fab fragments, including Fab or immunoglobulin expression library products.

An immunospecific antibody can be obtained by immunization of an animal with a polypeptide according to the invention and the recovery of the immunospecific antibody.

The invention also relates to an immunospecific antibody for a polypeptide according to the invention, such as defined previously.

A polypeptide according to the invention, one of its fragments, an analog, one of its variants or a cell expressing this polypeptide can also be used to produce immunospecific antibodies.

The term "immunospecific" means that the antibody possesses a better affinity for the polypeptide of the invention than for other polypeptides known in the prior art.

The immunospecific antibodies can be obtained by administration of a polypeptide of the invention, of one of its fragments, of an analog or of an epitopic fragment or of a cell expressing this polynucleotide in a mammal, preferably non human, according to method well known to a person skilled in the art.

For the preparation of monoclonal antibodies, typical methods for antibody production can be used, starting from cell lines, such as the hybridoma technique (Kohler et al., Nature (1975) 256: 495-497), the trioma technique, the human B cell hybridoma technique (Kozbor et al., Immunology Today (1983) 4:72) and the EBV hybridoma technique (Cole et al., MONOCLONAL ANTIBODIES AND CANCER THERAPY, pp. 77-96, Alan R. Liss, 1985).

The techniques of single chain antibody production such as described, for example, in US Patent N° 4,946, 778 can equally be used.

Transgenic animals such as mice, for example, can equally be used to produce humanized antibodies.

### Medications and treatments of diseases

The present invention also has for its object a medication containing as an active agent at least one polynucleotide, a recombinant vector, a host cell and/or an immunospecific antibody of the invention as defined above.

The invention also relates to the use of at least one polynucleotide, a recombinant vector, a host cell and/or an immunospecific antibody of the invention as defined above, for the preparation of a medication for the prevention or the treatment of a disorder or a disease for a patient having a deficiency in the synthesis of PGH, which deficiency is linked to the presence of a nucleotide sequence having at least 95% identity with the nucleotide sequence SEQ ID N° 1 in the genome of said patient.

Preferably, the invention concerns the use of a at least one polynucleotide, a recombinant vector, a host cell and/or an immunospecific antibody of the invention as defined above, for the preparation of a medication for the prevention or the treatment of one of the disorders or diseases involving the human growth and development, such as fetal growth and development, perinatal carbohydrate metabolism, phallic growth, craniofacial developments and the Laron type of dwarfism, secretion and stimulating of IGF-I, the lipid metabolism such as arteriosclerosis, angiogenesis, cancer such as breast cancer in premenopausal and prostate cancer and the immune system.

The present invention has also for its object the use of a growth hormone for the preparation of a medication for a patient having a deficiency in the synthesis of PGH, which deficiency is linked to the presence of a nucleotide sequence having at least 95% identity with the nucleotide sequence SEQ ID N° 1 in the genome of said patient

The growth hormone is preferably the placental growth hormone (PGH) and/or the adult growth hormone (GH).

The growth hormone is particularly useful for the prevention or the treatment of one of the disorders or diseases involving the human growth and development, such as fetal growth and development, perinatal carbohydrate metabolism, phallic growth, craniofacial developments and the Laron type of dwarfism, secretion and stimulating of IGF-I, the lipid metabolism such as arteriosclerosis, angiogenesis, cancer such as breast cancer in premenopausal and prostate cancer and the immune system.

The patient is preferably a fetus or a child preferably less than ten years old such as for example an infant just after birth.

Preferably, the nucleotide sequence having at least 95 % identity with the nucleotide sequence SEQ ID N° 1 is the nucleotide sequence SEQ ID N° 1.

The dosage of the compounds of the invention, useful as active agent, depends on the choice of the compound, the therapeutic indication, the mode of administration, the nature of the formulation, the nature of the subject and the judgment of the doctor.

When it is used as active agent, a polypeptide according to the invention is generally administered at doses ranging between 0.05 and 0.5 mg/kg of the subject, per week and according to the mode of administration.

The invention also has as an object a pharmaceutical composition that contains at least one polypeptide, a polynucleotide, a recombinant vector, a host cell and/or an immunospecific antibody of the invention as defined above.

In these pharmaceutical compositions, the active agent is advantageously present at physiologically effective doses.

These pharmaceutical compositions can be, for example, solids or liquids and be present in pharmaceutical forms currently used in human medicine, such as for example simple or coated tablets, gels, pellets, caramels, suppositories and preferably injectable preparations and powders for injectables. These pharmaceutical forms can be prepared according to typical methods.

The active agent(s) can be incorporated into typically employed excipients in these pharmaceutical compositions, such as talc, Arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, preservatives.

The active agent(s) according to the invention can be employed alone or in combination with other compounds, such as therapeutic compounds such as adult human growth hormone, interferons, even other cytokines such as interleukin, for example.

The different formulations of the pharmaceutical compositions are adapted according to the mode of administration.

The pharmaceutical compositions can be administered by different routes of administration known to a person skilled in the art.

The invention equally has for an object a diagnostic kit that contains at least one polypeptide, a polynucleotide, a recombinant vector, a host cell and/or an immunospecific antibody of the invention as defined above.

The appropriate excipients used in the diagnostic composition can be generally buffers and preservatives.

## Claims

1. Isolated polynucleotide comprising :
a) a nucleotide sequence having at least 95 % identity with one of the nucleotide sequences chosen in the group consisting of SEQ ID N° 1, SEQ ID N° 2, SEQ ID N° 3, SEQ ID N° 4, SEQ ID N° 5 and SEQ ID N° 6, or
b) a nucleotide sequence complementary to a nucleotide sequence under a).

2. Isolated polynucleotide comprising :
a) one of the nucleotide sequences chosen in the group consisting of SEQ ID N° 1, SEQ ID N° 2, SEQ ID N° 3, SEQ ID N° 4, SEQ ID N° 5 and SEQ ID N° 6, or
b) a nucleotide sequence complementary to a nucleotide sequence under a).

3. Polynucleotide according to any one of claims 1 and 2, **characterized in that** it consists of the nucleotide sequence SEQ ID N° 1.

4. Polynucleotide according to any one of claims 1 and 2, **characterized in that** it consists of the nucleotide coding sequence SEQ ID N° 2.

5. Polynucleotide according to any one of claims 1 and 2, **characterized in that** it consists of the nucleotide coding sequence SEQ ID N° 3.

6. Polynucleotide according to any one of claims 1 and 2, **characterized in that** it consists of the nucleotide coding sequence SEQ ID N° 4.

7. Polynucleotide according to any one of claims 1 and 2, **characterized in that** it consists of the nucleotide coding sequence SEQ ID N° 5.

8. Polynucleotide according to any one of claims 1 and 2, **characterized in that** it consists of the nucleotide coding sequence SEQ ID N° 6.

9. Isolated polynucleotide, **characterized in that** it encodes for a polypeptide consisting of the amino acid sequence SEQ ID N° 7.

10. Polynucleotide according to any one of claims 1 to 9, **characterized in that** it is composed of a DNA or RNA molecule.

11. Use of all or part of a polynucleotide according to any one of claims 1 to 10, to identify, hybridize and/or amplify :
- a polynucleotide consisting of one of the nucleotide sequences chosen from SEQ ID N° 1, SEQ ID N° 2, SEQ ID N° 3, SEQ ID N° 4, SEQ ID N° 5 and SEQ ID N° 6; or
- a part of a polynucleotide consisting of one of the nucleotide sequences chosen from SEQ ID N° 1, SEQ ID N° 2, SEQ ID N° 3, SEQ ID N° 4, SEQ ID N° 5 and SEQ ID N° 6, provided that said part comprises the SNP c2465a (or c51a).

12. Use of all or part of a polynucleotide according to any one of claims 1 to 10, as a genotyping tool in the nucleotide sequence having 90 to 100 % identity with the nucleotide sequence of a growth hormone gene, this use allows to determine whether said growth hormone gene comprises the SNP c2465a (or c51 a).

13. Process for determination of the frequency of the SNP c2465a (or c51a) in a polynucleotide according to any one of claims 1 to 10, in which a genotyping is performed in an individual or a population of individuals.

14. Process according to claim 13, in which the genotyping is carried out by minisequencing.

15. Process according to claim 14, in which the minisequencing is carried out with the sense and antisense primers corresponding respectively to the nucleotide sequences SEQ ID N° 8 and SEQ ID N° 9.

16. Recombinant vector comprising a polynucleotide according to any one of claims 1 to 10.

17. Host cell comprising a recombinant vector according to claim 16.

18. Process for preparation of a polypeptide, **characterized in that** a host cell according to claim 17 is cultivated in a culture medium and said polypeptide is isolated from the culture medium.

19. Isolated polypeptide comprising an amino acid sequence having at least 95 % identity with the amino acid sequence SEQ ID N° 7.

20. Polypeptide according to claim 19, **characterized in that** it comprises the amino acid sequence SEQ ID N° 7.

21. Polypeptide according to any one of claims 19 and 20, **characterized in that** it consists of the amino acid sequence SEQ ID N° 7.

22. Process for obtaining an immunospecific antibody, **characterized in that** it consists of the immunization of an animal with a polypeptide according to any one of claims 19 and 21, and the recovery of the immunospecific antibody.

23. Immunospecific antibody for a polypeptide according to any one of claims 19 and 21.

24. Medication comprising as an active agent at least one polynucleotide according to any one of claims 1 to 10, a recombinant vector according to claim 16, a host cell according to claim 17 and/or an immunospecific antibody according to claim 23.

25. Use of a polynucleotide according to any one of claims 1 to 10, a recombinant vector according to claim 16, a host cell according to claim 17 and/or an immunospecific antibody according to claim 23, for the preparation of a medication for the prevention or the treatment of a disorder or a disease for a patient having a deficiency in the synthesis of PGH, which deficiency is linked to the presence of a nucleotide sequence having at least 95% identity with the nucleotide sequence SEQ ID N° 1 in the genome of said patient.

26. Use according to claim 25, for the prevention or the treatment of one of the disorders or diseases selected from the group consisting of the disorders or diseases involving the human growth and development, such as fetal growth and development, perinatal carbohydrate metabolism, phallic growth, craniofacial developments and the Laron type of dwarfism, secretion and stimulating of IGF-I, the lipid metabolism such as arteriosclerosis, angiogenesis, cancer such as breast cancer in premenopausal and prostate cancer and the immune system.

27. Use of a growth hormone for the preparation of a medication for a patient having a deficiency in the synthesis of PGH, which deficiency is linked to the presence of a nucleotide sequence having at least 95% identity with the nucleotide sequence SEQ ID N° 1 in the genome of said patient.

28. Use according to claim 27, wherein said growth hormone is placental growth hormone (PGH)and/or adult growth hormone (GH).

29. Use according to any one of claims 27 and 28, for the prevention or the treatment of one of the disorders or diseases involving the human growth and development, such as fetal growth and development, perinatal carbohydrate metabolism, phallic growth, craniofacial developments and the Laron type of dwarfism, secretion and stimulating of IGF-I, the lipid metabolism such as arteriosclerosis, angiogenesis, cancer such as breast cancer in premenopausal and prostate cancer and the immune system.

30. Use according to any one of claims 27 and 29, wherein the patient is a fetus or a child.

31. Use according to any one of claims 25 to 30, wherein the nucleotide sequence having at least 95 % identity with the nucleotide sequence SEQ ID N° 1 is the nucleotide sequence SEQ ID N° 1.

32. Pharmaceutical composition containing as an active agent at least one polypeptide according to any one of claims 19 and 21, all or part of a polynucleotide according to any one of claims 1 to 10, a recombinant vector according to claim 16, a host cell according to claim 17 and/or an immunospecific antibody according to claim 23, as well as an appropriate pharmaceutically acceptable excipient.

33. Diagnostic kit comprising at least one polypeptide according to any one of claims 19 and 21, all or part of a polynucleotide according to any one of claims 1 to 10, a recombinant vector according to claim 16, a host cell according to claim 17 and/or an immunospecific antibody according to claim 23.
